(19) Europäisches Patentamt
European Patent Office
Office européen des brevets

(11) Veröffentlichungsnummer: **0 064 255**
**B1**

(12) # EUROPÄISCHE PATENTSCHRIFT

(45) Veröffentlichungstag der Patentschrift: **12.12.84**

(51) Int. Cl.³: **C 07 D 405/12**, A 61 K 31/445

(21) Anmeldenummer: **82103535.9**

(22) Anmeldetag: **27.04.82**

(54) **Benzopyranylether, Verfahren zu deren Herstellung sowie diese Verbindungen enthaltende Arzneimittel und Zwischenprodukte.**

(30) Priorität: **02.05.81 DE 3117389**

(43) Veröffentlichungstag der Anmeldung: **10.11.82 Patentblatt 82/45**

(45) Bekanntmachung des Hinweises auf die Patenterteilung: **12.12.84 Patentblatt 84/50**

(84) Benannte Vertragsstaaten: **AT BE CH DE FR GB IT LI LU NL SE**

(56) Entgegenhaltungen: **EP-A-0 013 894**

**Die Akte enthält technische Angaben, die nach dem Eingang der Anmeldung eingereicht wurden und die nicht in dieser Patentschrift enthalten sind.**

(73) Patentinhaber: **BOEHRINGER MANNHEIM GMBH, Patentabteilung, Abt. E Sandhofer Strasse 112-132 Postfach 31 01 20, D-6800 Mannheim 31 Waldhof (DE)**

(72) Erfinder: **Friebe, Walter-Gunar, Dr., Heidelberger Landstrasse 111d, D-6100 Darmstadt 13 (DE)**
Erfinder: **Kampe, Wolfgang, Dr. rer. nat., Zedernstrasse 49, D-6805 Heddesheim (DE)**
Erfinder: **Roesch, Egon, Dr. med., Werderstrasse 44, D-6800 Mannheim 1 (DE)**
Erfinder: **Wilhelms, Otto-Henning, Dr. rer. nat., Odenwaldstrasse 25/2, D-6941 Weinheim-Rittenweier (DE)**

EP 0 064 255 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Beschreibung

Die Erfindung betrifft neue Benzopyranderivate, Verfahren zu deren Herstellung, diese Verbindungen enthaltende Arzneimittel, sowie Zwischenprodukte.

Die neuen Verbindungen sowie ihre pharmakologisch unbedenklichen Salze wirken oral bereits in niedrigen Dosen hemmend auf anaphylaktische Reaktionen der Haut und des Bronchialsystems. Ein schwacher antiödematöser Effekt wurde beobachtet. Gleichzeitig antagonisieren sie auch durch Allergen freigesetzte Mediatoren, z. B. Histamin.

Verbindungen ähnlicher Konstitution sind in der DE-A-2 901 336 beansprucht. Es wurde nunmehr gefunden, daß auch Verbindungen, die an Stelle des Cumarinrestes einen 2-Methylchromonrest tragen, eine ausgeprägte antiallergische Wirkung zeigen.

Die Erfindung betrifft Benzopyranderivate der allgemeinen Formel I

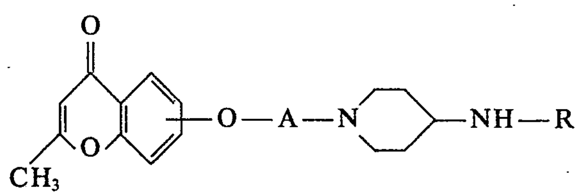 (I)

in welcher

A einen Alkylenrest mit 2 bis 4 Kohlenstoffatomen und

R eine $C_1-C_5$ Alkanoylgruppe, die durch Halogen oder Phenyl substituiert sein kann, eine Benzoyl- oder Naphthoylgruppe, die ein- oder mehrfach durch Halogen, Hydroxyl, $C_1-C_4$-Alkyl, $C_1-C_4$-Alkoxy, $C_1-C_4$-Alkoxycarbonyl, $C_1-C_5$-Alkanoyloxy, Carboxyl, Nitro, Amino, $C_1-C_5$-Alkanoylamino, Nitril, Trifluormethyl, Carbamoyl, $C_1-C_4$-Alkylthio, $C_1-C_4$-Alkylsulfinyl, $C_1-C_4$-Alkylsulfonyl, $C_1-C_5$-Alkanoyl, Benzoyl, Hydroxy-$C_1-C_4$-Alkyl oder $C_1-C_4$-Alkoxy-$C_1-C_4$-Alkyl substituiert sein kann, eine Furancarbonyl-, Thiophencarbonyl-, Pyridincarbonyl-, Tetrahydrofurancarbonyl- oder Tetrahydrothiophencarbonyl-Gruppe oder einen $C_3-C_7$-Cycloalkylcarbonyl-Rest

bedeuten,
sowie deren Salze mit pharmakologisch verträglichen Säuren.

Weiterhin betrifft die Erfindung pharmazeutische Präparate mit einem Gehalt an Verbindungen der allgemeinen Formel I sowie die Verwendung von Verbindungen der allgemeinen Formel I zur Herstellung solcher Präparate.

Die für A stehenden Alkylenreste können geradkettig oder verzweigt sein. Bevorzugt ist der Trimethylenrest.

Die $C_1-C_4$-Alkyl-Reste in den genannten Gruppen können geradkettig oder verzweigt sein. Bevorzugt ist der Methyl-Rest.

Als Halogenatome kommen Fluor, Chlor und Brom in Frage.

Außer den in den Beispielen genannten Verbindungen sind Gegenstand der Erfindung insbesondere alle Substanzen, die jede mögliche Kombination der in den Beispielen genannten Substituenten aufweisen.

Der Ether-Substituent des Benzopyranringes kann in 5-, 6- oder 7-Stellung stehen, bevorzugt ist die 7-Position.

Die Verbindungen der allgemeinen Formeln I und Ia werden hergestellt, indem man in an sich bekannter Weise eine Verbindung der allgemeinen Formel II

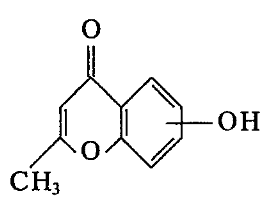 (II)

mit einer Verbindung der allgemeinen Formel III

$$X-A-Y \quad (III)$$

in der

X und Y reaktive Reste bedeuten und A die obengenannte Bedeutung hat,

und einer Verbindung der allgemeinen Formel IV

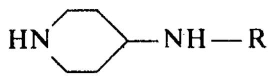

(IV)

in der R die obengenannte Bedeutung hat,
umsetzt,
anschließend gewünschtenfalls die Gruppe R durch bekannte Verfahren in eine andere, der obengenannten Bedeutung entsprechende Gruppe R
umwandelt
und das so erhaltene Reaktionsprodukt gewünschtenfalls in ein pharmakologisch verträgliches Salz überführt.

Als reaktive Reste X und Y der Verbindungen der allgemeinen Formel III kommen Chlor, Brom, Mesyloxy und Tosyloxy in Frage.

Das erfindungsgemäße Verfahren führt man beispielsweise so durch, daß man zunächst Verbindungen der allgemeinen Formel III mit Verbindungen der allgemeinen Formel IV kondensiert und das so erhaltene Reaktionsprodukt isoliert. Dieses Zwischenprodukt wird dann mit einer Verbindung der allgemeinen Formel II zur Reaktion gebracht. Die Umsetzung erfolgt zweckmäßig in alkalischem Medium, beispielsweise in einem niederen Alkohol wie z. B. Isopropanol in Anwesenheit von Natriumisopropanolat oder in Dimethylformamid in Anwesenheit von Kaliumcarbonat.

Eine andere Variante besteht darin, zunächst Verbindungen der allgemeinen Formel II mit Verbindungen der allgemeinen Formel III zur Reaktion zu bringen; anschließend wird das so erhaltene Reaktionsgemisch mit Verbindungen der allgemeinen Formel IV zum gewünschten Endprodukt der allgemeinen Formeln I oder Ia umgesetzt.

Eine nachträgliche Umwandlung der Gruppe R in der allgemeinen Formel I in eine andere Gruppe R erfolgt beispielsweise durch Acylierung einer Verbindung der allgemeinen Formel I, in der R für Wasserstoff steht, mit einer Verbindung R—Z, wobei Z einen reaktiven Rest darstellt. Verbindungen der allgemeinen Formel I mit der Bedeutung R = Wasserstoff stellen somit wertvolle Zwischenprodukte zur Herstellung anderer Verbindungen der allgemeinen Formel I dar. Reaktive Reste Z können alle Reste sein, die in der Peptidchemie zur Aktivierung von Carbonsäuren Verwendung finden, beispielsweise Halogenatome, die Azidogruppe, Alkyloxy-, Aryloxy- und Acyloxy-Gruppen.

Gegenstand der Erfindung sind demnach auch Benzopyranylether der allgemeinen Formel Ia

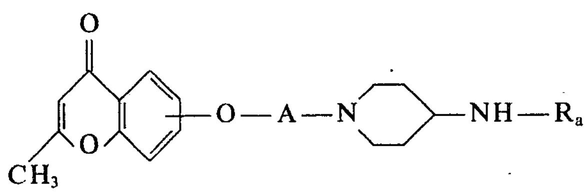

(Ia)

in welcher

A einen Alkylrest mit 2 bis 4 Kohlenstoffatomen und
$R_a$ Wasserstoff

bedeuten,
sowie deren Salze.

Eine weitere Möglichkeit der nachträglichen Umwandlung der Gruppe R in der allgemeinen Formel I besteht darin, daß man nach allgemein bekannten Verfahren einen oder mehrere Substituenten des Acylrestes R beispielsweise durch Veresterung, Verseifung, Reduktion, Alkylierung, Acylierung, Hydrogenolyse, Oxidation, Amidierung oder Eliminierung in einen oder mehrere andere Substituenten des Acylrestes R umwandelt.

Die Ausgangsverbindungen der allgemeinen Formeln II, III und IV sind literaturbekannte Substanzen oder können in Analogie zu literaturbekannten Verfahren hergestellt werden.

Die pharmakologisch verträglichen Salze erhält man in üblicher Weise, z. B. durch Neutralisation der Verbindungen der allgemeinen Formel I mit nichttoxischen anorganischen oder organischen Säuren wie z. B. Salzsäure, Schwefelsäure, Phosphorsäure, Bromwasserstoffsäure, Essigsäure, Milchsäure, Citronensäure, Apfelsäure, Salicylsäure, Malonsäure, Maleinsäure oder Bernsteinsäure.

Zur Herstellung von Arzneimitteln werden die Verbindungen der allgemeinen Formel I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z. B. Olivenöl, suspendiert oder gelöst.

Die Substanzen der allgemeinen Formel I können in flüssiger oder fester Form oral und parenteral appliziert werden. Als Injektionsmedium kommt vorzugsweise Wasser zur Anwendung, welches die

bei Injektionslösungen üblichen Stabilisierungsmittel, Lösungsvermittler und/oder Puffer enthält. Derartige Zusätze sind z. B. Tartrat- oder Borat-Puffer, Ethanol, Dimethylsulfoxid, Komplexbildner (wie Ethylendiamintetraessigsäure), hochmolekulare Polymere (wie flüssiges Polyethylenoxid) zur Viskositätsregulierung oder Polyethylen-Derivate von Sorbitanhydriden.

Feste Trägerstoffe sind z. B. Stärke, Lactose, Mannit, Methylcellulose, Talkum, hochdisperse Kieselsäure, höhermolekulare Polymere (wie Polyethylenglykole).

Für die orale Applikation geeignete Zubereitungen können gewünschtenfalls Geschmacks- und Süßstoffe enthalten. Für die äußerliche Anwendung können die erfindungsgemäßen Substanzen I auch in Form von Pudern und Salben verwendet werden. Sie werden dazu z. B. mit pulverförmigen, physiologisch verträglichen Verdünnungsmitteln bzw. üblichen Salbengrundlagen vermischt.

Die verabreichte Dosierung hängt vom Alter, der Gesundheit und dem Gewicht des Empfängers, dem Ausmaß der Krankheit, der Art gleichzeitiger gegebenenfalls durchgeführter weiterer Behandlungen, der Häufigkeit der Behandlungen und der Art der gewünschten Wirkung ab. Üblicherweise beträgt die tägliche Dosis der aktiven Verbindung 0,1 bis 50 mg/kg Körpergewicht. Normalerweise sind 0,5 bis 40 und vorzugsweise 1,0 bis 20 mg/kg/Tag in einer oder mehreren Anwendungen pro Tag wirksam, um die gewünschten Resultate zu erhalten.

Außer den in den nachstehenden Beispielen genannten Substanzen sind die folgenden Verbindungen bevorzugt:

6-[3-(4-Benzamido-piperidino)-propoxy]-2-methyl-4H-1-benzopyran-4-on
7-(3-[4-(Pyridin-3-carboxamido)-piperidino]-propoxy)-2-methyl-4H-1-benzopyran-4-on
7-(3-[4-(2-Chlor-benzamido)-piperidino]-propoxy)-2-methyl-4H-1-benzopyran-4-on
7-[2-(4-Benzamido-piperidino)-ethoxy]-2-methyl-4H-1-benzopyran-4-on
7-[4-(4-α-Naphthoylamino-piperidino)-butoxy]-2-methyl-4H-1-benzopyran-4-on
7-[3-(4-Trifluoracetamido-piperidino)-propoxy]-2-methyl-4H-1-benzopyran-4-on

## Beispiel 1

### 7-[3-(4-Benzamido-piperidino)-propoxy] 2-methyl-4-H-1 benzopyran-4-on

Zu der Lösung von 0,69 g (0,03 mol) Natrium in 75 ml 2-Propanol gibt man 5,3 g (0,03 mol) 7-Hydroxy-2-methyl-4H-1-benzopyran-4-on, erwärmt 10 min zum Rückfluß, fügt 8,4 g (0,03 mol) 3-(4-Benzamido piperidino)-propylchlorid, gelöst in 25 ml 2-Propanol, zu, erhitzt 12 Stunden zum Rückfluß, engt ein, nimmt in Dichlormethan auf, wäscht mit verd. Natronlauge, engt die organische Phase ein und kristallisiert den Rückstand aus 2-Propanol um.

Man isoliert 6,8 g Titelverbindung (54% d. Th.) vom Schmp. 173—175°C.

## Beispiel 2

In analoger Weise wie in Beispiel 1 beschrieben erhält man:

| Bezeichnung | Ausbeute % | Schmelzpunkt °C (Lösungsmittel) |
|---|---|---|
| 5-[3-(4-Benzamido-piperidino)-propoxy]-2-methyl-4H-1-benzopyran-4-on aus 5-Hydroxy-2-methyl-4H-1-benzopyran-4-on und 3-(4-Benzamido-piperidino)-propylchlorid | 38 | 193—194 (2-Propanol) |

## Beispiel 3

### 7-[3-(4-Phenylacetamido-piperidino)-propoxy]-2-methyl-4H-1-benzopyran-4-on

Eine Mischung aus 5,94 g (0,02 mol) 7-(3-Brom propoxy)-2-methyl-4H-1-benzopyran-4-on, 100 ml Tetrahydrofuran, 13 ml Triethylamin und 4,4 g (0,02 mol) 4-Phenylacetamidopiperidin wird 6 Stunden zum Rückfluß erhitzt, eingeengt, mit Wasser versetzt, mit Dichlormethan extrahiert, der Extrakt eingeengt und aus 2-Propanol umkristallisiert.

Es verbleiben 5,0 g Titelverbindung (58% d. Th.) vom Schmp. 172—173°C.

Beispiel 4

In analoger Weise wie in Beispiel 3 beschrieben erhält man:

| | Bezeichnung | Ausbeute % | Schmelzpunkt °C (Lösungsmittel) |
|---|---|---|---|
| a) | 7-[3-(4-Acetamido-piperidino)-propoxy]-2-methyl-4H-1-benzopyran-4-on aus 7-(3-Brom-propoxy)-2-methyl-4H-1-benzopyran-4-on und 4-Acetamido-piperidin | 39 | 165—167 (Aceton) |
| b) | 7-⟨3-[4-(4-Fluor-benzamido)-piperidino]-propoxy⟩-2-methyl-4H-1-benzopyran-4-on aus 7-(3-Brom-propoxy)-2-methyl-4H-1-benzopyran-4-on und 4-(4-Fluor-benzamido)-piperidin | 59 | 188—189 (2-Propanol) |
| c) | 7-⟨3-[4-(2-Methyl-benzamido)-piperidino]-propoxy⟩-2-Methyl-4H-1-benzopyran-4-on aus 7-(3-Brom-propoxy)-2-methyl-4H-1-benzopyran-4-on und 4-(2-Methyl-benzamido)-piperidin | 45 | Oxalat 205—207 (Methanol/Ether) |
| d) | 7-⟨3-[4-(2-Methoxy-benzamido)-piperidino]-propoxy⟩-2-methyl-4H-1-benzopyran-4-on aus 7-(3-Brom-propoxy)-2-methyl-4H-1-benzopyran-4-on und 4-(2-Methoxy-benzamido)-piperidin | 33 | Oxalat 204—206 (Ethanol) |
| e) | 7-⟨3-[4-(2-Amino-benzamido)-piperidino]-propoxy⟩-2-methyl-4H-1-benzopyran-4-on aus 7-(3-Brom-propoxy)-2-methyl-4H-1-benzopyran-4-on und 4-(2-Amino-benzamido)-piperidin | 35 | 154—157 (2-Propanol) |
| f) | 7-⟨3-[4-(Tetrahydrofuran-2-carboxamido) piperidino]-propoxy⟩-2-methyl-4H-1-benzopyran-4-on aus 7-(3-Brom-propoxy)-2-methyl-4H-1-benzopyran-4-on und 4-(Tetrahydrofuran-2-carboxamido)-piperidin | 21 | 105—107 (Ethylacetat/Ligroin) |
| g) | 7-[3-(4-Amino-piperidino)-propoxy]-2-methyl-4H-1-benzopyran-4-on aus 7-(3-Brom-propoxy)-2-methyl-4H-1-benzopyran-4-on und 4-Amino-piperidin | 79 | 113—115 (Dichlormethanol) |

Beispiel 5

7-⟨3-[4-(2-Nitro-benzamido)-piperidino]-propoxy⟩-2-methyl-4H-1-benzopyran-4-on

Zu einer Mischung aus 6,95 g (0,022 mol) 7-[3-(4-Amino-piperidino)-propoxy]-2-methyl-4H-1-benzopyran-4-on (Verbindung aus Beispiel 4 g), 4,2 g (0,05 mol) Natriumhydrogencarbonat und 50 ml Dichlormethan tropft man die Lösung von 5,1 g (0,027 mol) 2-Nitro-benzoylchlorid in 25 ml Dichlormethan, erwärmt 6 Stunden zum Rückfluß, wäscht mit Wasser und engt ein. Nach Umkristallisation aus 2-Propanol erhält man 4,0 g Titelverbindung (40% d. Th.) vom Schmp. 185—187° C.

Beispiel 6

In analoger Weise wie in Beispiel 5 beschrieben erhält man:

| | Bezeichnung | Ausbeute % | Schmelzpunkt °C (Lösungsmittel) |
|---|---|---|---|
| a) | 7-[3-(4-Cyclopropancarboxamido-piperidino)-propoxy]-2-methyl-4H-1-benzopyran-4-on aus 7-[3-(4-Amino-piperidino)-propoxy]-2-methyl-4H-1-benzopyran-4-on und Cyclopropancarbonylchlorid | 30 | 170–172 (Ethylacetat) |
| b) | 7-(3-[4-(Thiophen-2-carboxamido)-piperidino]-propoxy)-2-methyl-4H-1-benzopyran-4-on aus 7-[3-(4-Amino-piperidino)-propoxy]-2-methyl-4H-1-benzopyran-4-on und Thiophen-2-carbonylchlorid | 38 | 178–190 (Dichlormethan/Methanol) |

Beispiel 7

7-(3-[4-(2-Hydroxy-benzamido)-piperidino]-propoxy)-2-methyl-4H-1-benzopyran-4-on

Eine Mischung aus 4,9 g (0,02 mol) 4-(2-Acetoxy-benzamido)-piperidin, 5,6 g (0,02 mol) 7-(3-Brom-propoxy)-2-methyl-4H-1-benzopyran-4-on, 13,9 ml (0,19 mol) Triethylamin und 100 ml Tetrahydrofuran wird 10 Stunden zum Rückfluß erhitzt und eingeengt. Das als Rohprodukt erhaltene 7-(3-[4-(2-Acetoxy-benzamido)-piperidino]-propoxy)-2-methyl-4H-1-benzopyran-4-on versetzt man mit 100 ml N Natronlauge, rührt 1 Stunde, filtriert, wäscht mit Ethylacetat und stellt die wäßrige Phase neutral. Den Niederschlag nimmt man in Aceton auf, versetzt mit überschüssiger etherischer Chlorwasserstoffsäure und isoliert das Dihydrochlorid der Titelverbindung (4,6 g 45% d. Th.) vom Schmp. 169–170°C.

Beispiel 8

7-[3-(4-Benzamido-piperidino)-propoxy] 2-methyl-4-H-1 benzopyran-4-on

Es wurden Tabletten hergestellt: Jede Tablette enthält 10 mg 7-[3 (4-Benzamido-piperidino) pro poxy]-2-methyl-4-H-1-benzopyran-4-on. Die Tabletten wurden gemäß der folgenden Rezeptur herge stellt:

| | |
|---|---|
| 7-[3-(4-Benzamido-piperidino)-propoxyl-2-methyl-4-H-1-benzopyran-4-on | 10 g |
| Lactose | 80 g |
| Stärke | 29 g |
| Magnesiumstearat | 1 g |

Der Wirkstoff wurde fein pulverisiert und mit Lactose und Stärke vermischt. Das Gemisch wurde in herkömmlicher Weise granuliert. Magnesiumstearat wurde zu dem Granulat gegeben und das Gemisch zu 1000 Tabletten mit einem Einzelgewicht von 0,12 g verpreßt.

**Patentansprüche**

1. Benzopyranylether der allgemeinen Formel I

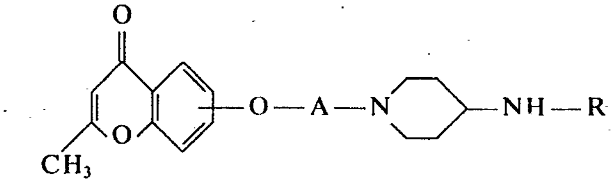

(I)

in welcher

A einen Alkylenrest mit 2 bis 4 Kohlenstoffatomen und

R eine $C_1$—$C_5$ Alkanoylgruppe, die durch Halogen oder Phenyl substituiert sein kann, eine Benzoyl- oder Naphthoylgruppe, die ein- oder mehrfach durch Halogen, Hydroxyl, $C_1$—$C_4$-Alkyl, $C_1$—$C_4$-Alkoxy, $C_1$—$C_4$-Alkoxycarbonyl, $C_1$—$C_5$-Alkanoyloxy, Carboxyl, Nitro, Amino, $C_1$—$C_5$-Alkanoylamino, Nitril, Trifluormethyl, Carbamoyl, $C_1$—$C_4$-Alkylthio, $C_1$—$C_4$-Alkylsulfinyl, $C_1$—$C_4$-Alkylsulfonyl, $C_1$—$C_5$-Alkanoyl, Benzoyl, Hydroxy-$C_1$—$C_4$-Alkyl oder $C_1$—$C_4$-Alkyl substituiert sein kann, eine Furancarbonyl-, Thiophencarbonyl-, Pyridincarbonyl-, Tetrahydrofurancarbonyl- oder Tetrahydrothiophencarbonyl-Gruppe oder einen $C_3$—$C_7$-Cycloalkylcarbonyl-Rest

bedeuten, sowie deren Salze mit pharmakologisch verträglichen Säuren.

2. Benzopyranylether der allgemeinen Formel Ia

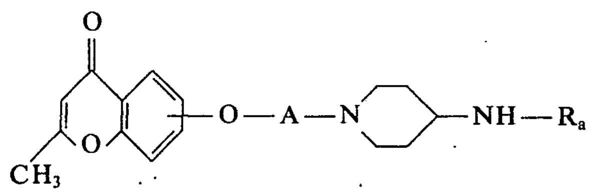

(Ia)

in welcher

A einen Alkylenrest mit 2 bis 4 Kohlenstoffatomen und

$R_a$ Wasserstoff

bedeuten, sowie deren Salze.

3. Verfahren zur Herstellung von Benzopyranylethern der allgemeinen Formel I'

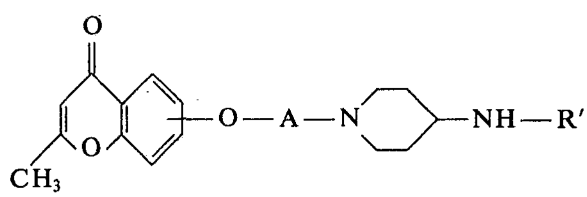

(I')

in welcher

A einen Alkylenrest mit 2 bis 4 Kohlenstoffatomen und

R' Wasserstoff, eine $C_1$—$C_5$-Alkanoylgruppe, die durch Halogen oder Phenyl substituiert sein kann, eine Benzoyl- oder Naphthoylgruppe, die ein- oder mehrfach durch Halogen, Hydroxyl, $C_1$—$C_4$-Alkyl, $C_1$—$C_4$-Alkoxy, $C_1$—$C_4$-Alkoxycarbonyl, $C_1$—$C_5$-Alkanoyloxy, Carboxyl, Nitro, Amino, $C_1$—$C_5$-Alkanoylamino, Nitril, Trifluormethyl, Carbamoyl, $C_1$—$C_4$-Alkylthio, $C_1$—$C_4$-Alkylsulfinyl, $C_1$—$C_4$-Alkylsulfonyl, $C_1$—$C_5$-Alkanoyl, Benzoyl, Hydroxy-$C_1$—$C_4$-Alkyl oder $C_1$—$C_4$-Alkoxy-$C_1$—$C_4$-Alkyl substituiert sein kann, eine Furancarbonyl, Thiophencarbonyl-, Pyridincarbonyl-, Tetrahydrofurancarbonyl- oder Tetrahydrothiophencarbonyl-Gruppe oder einen $C_3$—$C_7$-Cycloalkylencarbonyl-Rest

bedeuten, sowie deren Salze mit pharmakologisch verträglichen Säuren, dadurch gekennzeichnet, daß man in an sich bekannter Weise eine Verbindung der allgemeinen Formel II

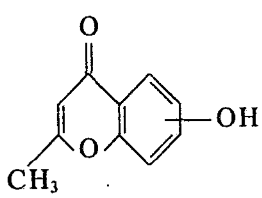

(II)

mit einer Verbindung der allgemeinen Formel III

X—A—Y (III)

in der

X und Y reaktive Reste bedeuten und A die obengenannte Bedeutung hat, und einer Verbindung der allgemeinen Formel IV

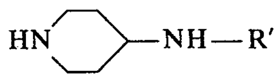 (IV)

in der R′ die obengenannte Bedeutung hat, umsetzt, anschließend gewünschtenfalls die Gruppe R′ durch bekannte Verfahren in eine andere, der obengenannten Bedeutung entsprechende Gruppe R′ umwandelt und das so erhaltene Reaktionsprodukt gewünschtenfalls in ein pharmakologisch verträgliches Salz überführt.

4. Verfahren zur Herstellung von Benzopyranylethern der allgemeinen Formel I

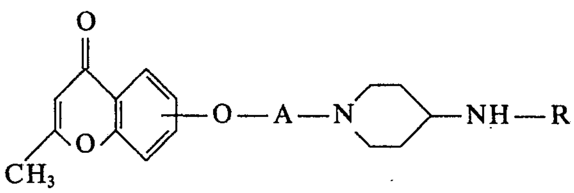 (I)

in welcher

A einen Alkylenrest mit 2 bis 4 Kohlenstoffatomen und

R eine $C_1-C_5$-Alkanoylgruppe, die durch Halogen oder Phenyl substituiert sein kann, eine Benzoyl- oder Naphthoylgruppe, die ein- oder mehrfach durch Halogen, Hydroxyl, $C_1-C_4$-Alkyl, $C_1-C_4$-Alkoxy, $C_1-C_4$-Alkoxycarbonyl, $C_1-C_5$-Alkanoyloxy, Carboxyl, Nitro, Amino, $C_1-C_5$-Alkanoylamino, Nitril, Trifluormethyl, Carbamoyl, $C_1-C_4$-Alkylthio, $C_1-C_4$-Alkylsulfinyl, $C_1-C_4$-Alkylsulfonyl, $C_1-C_5$-Alkanoyl, Benzoyl, Hydroxy-$C_1-C_4$-Alkyl oder $C_1-C_4$-Alkoxy-$C_1-C_4$-Alkyl substituiert sein kann, eine Furancarbonyl-, Thiophencarbonyl-, Pyridincarbonyl-, Tetrahydrofurancarbonyl- oder Tetrahydrothiophencarbonyl-Gruppe oder einen $C_3-C_7$-Cycloalkylcarbonyl-Rest

bedeuten, dadurch gekennzeichnet, daß man eine Verbindung der allgemeinen Formel Ia

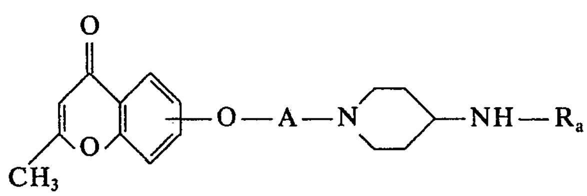 (Ia)

in welcher

A einen Alkylenrest mit 2 bis 4 Kohlenstoffatomen und

$R_a$ Wasserstoff

bedeuten, mit einer Verbindung R—Z, in der R die obige Bedeutung hat und Z einen reaktiven Rest darstellt, acyliert, anschließend gewünschtenfalls die Gruppe R durch bekannte Verfahren in eine andere, der obengenannten Bedeutung entsprechende Gruppe R umwandelt und das so erhaltene Reaktionsprodukt gewünschtenfalls in ein pharmakologisch verträgliches Salz

überführt.

5. Arzneimittel, gekennzeichnet durch einen Gehalt an Verbindungen gemäß Anspruch 1 und üblichen Träger- und Hilfsstoffen.

6. Verwendung von Verbindungen gemäß Anspruch 1 zur Herstellung von Arzneimitteln mit antiallergischer Eigenschaften.

7. Verbindungen gemäß Anspruch 1 zur Heilung von allergischen Krankheiten.

**Claims**

1. Benzopyranyl ethers of the general formula I

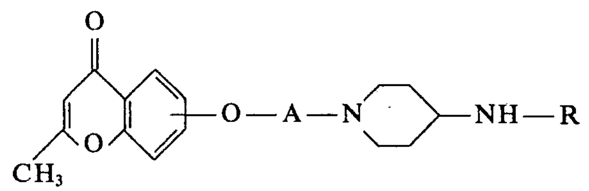

(I)

in which

A signifies an alkylene radical with 2 to 4 carbon atoms and
R a $C_1-C_5$-alkanoyl group which can be substituted by halogen or phenyl;
a benzoyl or naphthoyl group which can be substituted one or more times by halogen, hydroxyl, $C_1-C_4$-alkyl, $C_1-C_4$-alkoxy, $C_1-C_4$-alkoxycarbonyl, $C_1-C_5$-alkanoyloxy, carboxyl, nitro, amino, $C_1-C_5$-alkanoylamino, nitrile, trifluoromethyl, carbamoyl, $C_1-C_4$-alkylthio, $C_1-C_4$-alkylsulphinyl, $C_1-C_4$-alkylsulphonyl, $C_1-C_5$-alkanoyl, benzoyl, hydroxy-$C_1-C_4$-alkyl or $C_1-C_4$-alkoxy-$C_1-C_4$-alkyl;
a furancarbonyl, thiophenecarbonyl, pyridinecarbonyl, tetrahydrofurancarbonyl or tetrahydrothiophenecarbonyl group;
or a $C_3-C_7$-cycloalkylcarbonyl radical:

as well as their salts with pharmacologically acceptable acids.

2. Benzopyranyl ehters of the general formula Ia

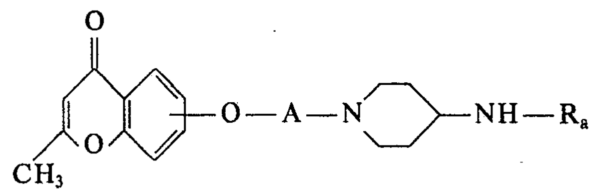

(Ia)

in which

A signifies an alkylene radical with 2 to 4 carbon atoms and
$R_a$ hydrogen, as well as their salts.

3. Process for the preparation of benzopyranyl ethers of the general formula I'

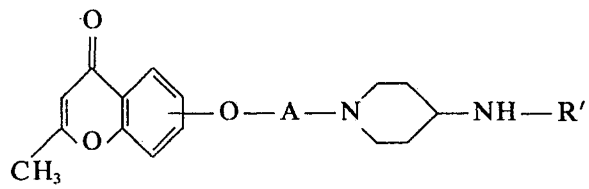

(I')

in which

A signifies an alkylene radical with 2 to 4 carbon atoms and
R' hydrogen, a $C_1-C_5$-alkanoyl group, which can be substituted by halogen or phenyl;
a benzoyl or naphthoyl group which can be substituted one or more times by halogen, hydroxyl, $C_1-C_4$-alkyl, $C_1-C_4$-alkoxy, $C_1-C_4$-alkoxycarbonyl, $C_1-C_5$-alkanoyloxy, carboxyl, nitro, amino, $C_1-C_5$-alkanoylamino, nitrile, trifluoromethyl, carbamoyl, $C_1-C_4$-alkylthio, $C_1-C_4$-alkylsulphinyl, $C_1-C_4$-alkylsulphonyl, $C_1-C_5$-alkanoyl, benzoyl, hydroxy-$C_1-C_4$-alkyl or $C_1-C_4$-alkoxy-$C_1-C_4$-alkyl; a furancarbonyl, thiophenecarbonyl, pyridinecarbonyl, tetrahydrofurancarbonyl or tetra-

hydrothiophenecarbonyl group;
or a $C_3-C_7$-cycloalkylcarbonyl radical, as well as of their salts with pharmacologically acceptable acids, characterised in that one reacts a compound of the general formula II

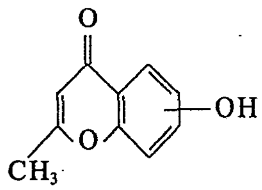

(II)

with a compound of the general formula III

X—A—Y (III)

in which

X and Y signify reactive residues and A has the above-mentioned meaning,
and a compound of general formula IV

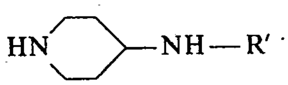

(IV)

in which R′ has the above-mentioned meaning, and subsequently, if desired, converts the group R′ by known processes into another group R′ corresponding to the above-mentioned meaning, and if desired converts the reaction product so obtained into a pharmacologically acceptable salt.

4. Process for the production of benzopyranyl ethers of the general formula I

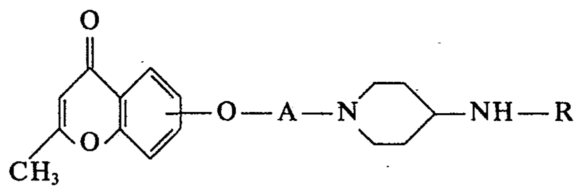

(I)

in which

A signifies an alkylene radical with 2 to 4 carbon atoms and
R a $C_1-C_5$-alkanoyl group which can be substituted by halogen or phenyl;
a benzoyl or naphthoyl group which can be substituted one or more times by halogen, hydroxyl, $C_1-C_4$-alkyl, $C_1-C_4$-alkoxy, $C_1-C_4$-alkoxycarbonyl, $C_1-C_5$-alkanoyloxy, carboxyl, nitro, amino, $C_1-C_5$-alkanoylamino, nitrile, trifluoromethyl, carbamoyl, $C_1-C_4$-alkylthio, $C_1-C_4$-alkylsulphinyl, $C_1-C_4$-alkylsulphonyl, $C_1-C_5$-alkanoyl, benzoyl, hydroxy-$C_1-C_4$-alkyl or $C_1-C_4$-alkoxy-$C_1-C_4$ -alkyl;
a furancarbonyl, thiophenecarbonyl, pyridinecarbonyl, tetrahydrofurancarbonyl or tetrahydrothiophenecarbonyl group;
or a $C_3-C_7$-cycloalkylcarbonyl radical, characterised in that one acylates a compound of the general formula Ia

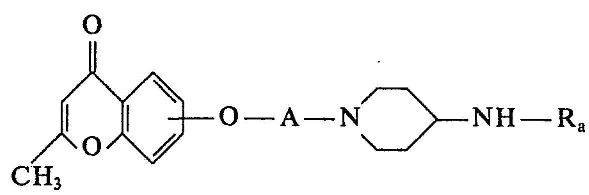

(Ia)

in which

A signifies an alkylene radical with 2 to 4 carbon atoms and
$R_a$ hydrogen,
with a compound R—Z,
in which R has the above meaning and Z represents a reactive residue, subsequently, if desired, converts the group R by known processes into another group R corresponding to the above-mentioned meaning and, if desired, converts the reaction product so obtained into a pharmacologically acceptable salt.

5. Medicaments, characterised by a content of compounds according to claim 1 and conventional carrier and adjuvant materials.

6. Use of compounds according to claim 1 for the preparation of medicaments with anti-allergic properties.

7. Compounds according to claim 1 for the healing of allergic diseases.

**Revendications**

1. Ethers benzopyranyliques de formule générale I

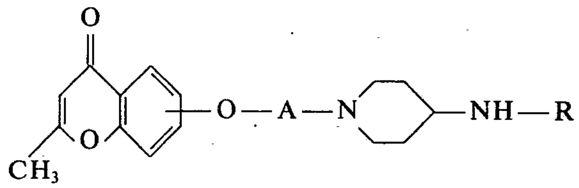

(I)

dans laquelle

A est un reste alkylène ayant de 2 à 4 atomes de carbone et
R est un groupe alcanoyle $C_1-C_5$ pouvant être substitué par de l'halogène ou du phényle, un groupe benzoyle ou naphthoyle pouvant être substitué une ou plusieurs foix par de l'halogène, de l'hydroxyle, de l'alkyle $C_1-C_4$, de l'alcoxy $C_1-C_4$, de l'alcoxycarbonyle $C_1-C_4$, de l'alcanoyloxy $C_1-C_5$, du carboxyle, du nitro, de l'amino, de l'alcanoylamino $C_1-C_5$, du nitrile, du trifluorométhyle, du carbamoyle, de l'alkylthio $C_1-C_4$, de l'alkylsulfinyle $C_1-C_4$, de l'alkylsulfonyle $C_1-C_4$, de l'alcanoyle $C_1-C_5$, du benzoyle, de l'hydroxy-alkyle $C_1-C_4$ ou de l'alcoxy $C_1-C_4$-alkyle-$C_1-C_4$, un groupe furanne-carbonyle, thiophènecarbonyle, pyridinecarbonyle, tétrahydrofurannecarbonyle ou tétrahydrothiophènecarbonyle, ou un reste cyclo-alkylcarbonyle $C_3-C_7$,

ainsi que leurs sels formés avec des acides pharmacologiquement compatibles.

2. Ethers benzopyranyliques de formule générale Ia

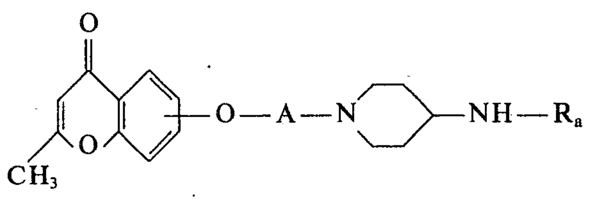

(Ia)

dans laquelle

A est un reste alkylène ayant de 2 à 4 atomes de carbone et
$R_a$ est de l'hydrogène,

ainsi que leurs sels.

3. Procédé de préparation d'éthers benzopyranyliques de formule générale I'

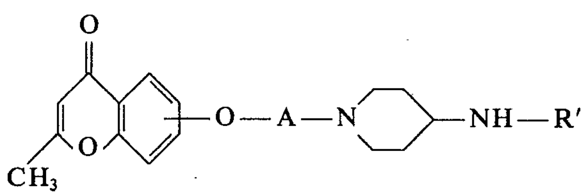

(I')

dans laquelle

A est un reste alkylène ayant de 2 à 4 atomes de carbone et
R' est de l'hydrogène un groupe alcanoyle $C_1-C_5$, pouvant être substitué par de l'halogène ou du phényle, un groupe benzoyle ou naphthoyle pouvant être substitué une ou plusieurs fois par de l'halogène, de l'hydroxyle, de l'alkyle $C_1-C_4$, de l'alcoxy $C_1-C_4$, de l'alcoxycarbonyle $C_1-C_4$, de l'alcanoyloxy $C_1-C_5$, du carboxyle, du nitro, de l'amino, de l'alcanoylamino $C_1-C_5$, du nitrile, du trifluorométhyle, du carbamoyle, de l'alkylthio $C_1-C_4$, de l'alkylsulfinyle $C_1-C_4$, de l'alkylsulfonyle $C_1-C_4$, de

l'alcanoyle $C_1-C_5$, du benzoyle, de l'hydroxyalkyle $C_1-C_4$ ou de l'alcoxy $C_1-C_4$-alkyle-$C_1-C_4$,
un groupe furanne-carbonyle, thiophènecarbonyle, pyridinecarbonyle, tétrahydrofurannecarbonyle ou tétrahydrothiophénecarbonyle, ou
un reste cyclo-alkylcarbonyle $C_3-C_7$

ainsi que leurs sels formés avec des acides pharmacologiquement compatibles,
caractérisé en ce que de façon en soi connue on fait réagir un composé de formule générale II

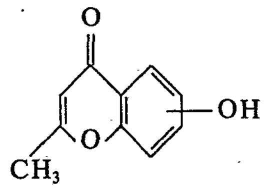
(II)

avec une composé de formule générale III

X—A—Y (III)

dans laquelle
X et Y sont des restes réactifs et A a la signification indiquée ci-dessus,
et avec un composé de formule générale IV

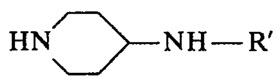
(IV)

dans laquelle R' a la signification donnée ci-dessus,
et en ce qu'ensuite on transforme, si on le souhaite, le groupe R' au moyen d'un procédé connu en un autre groupe R' correspondant à la signification ci-dessus donnée et
on transforme le produit de réaction ainsi obtenu si on le souhaite, en un sel pharmacologiquement compatible.

4. Procédé de préparation d'éthers benzopyranyliques de formule générale I

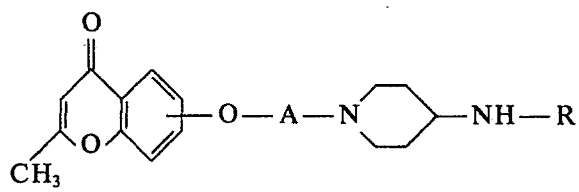
(I)

dans laquelle

A est un reste alkylène ayant de 2 à 4 atomes de carbone, et
R est un groupe alcanoyle $C_1-C_5$; pouvant être substitué par de l'halogène ou du phényle
un groupe benzoyle ou naphthoyle pouvant être substitué une ou plusieurs fois par de l'halogène, de l'hydroxyle, de l'alkyle $C_1-C_4$, de l'alcoxy $C_1-C_4$, de l'alcoxycarbonyle $C_1-C_4$, de l'alcanoyloxy $C_1-C_5$, du carboxyle, du nitro, de l'amino, de l'alcanoylamino $C_1-C_5$, du nitrile, du trifluorométhyle, di carbamoyle, de l'alkylthio $C_1-C_4$, de l'alkylsulfinyle $C_1-C_4$, de l'alkylsulfonyle $C_1-C_4$, de l'alcanoyle $C_1-C_5$, du benzoyle, de l'hydroxyalkyle $C_1-C_4$ ou de l'alcoxy $C_1-C_4$-alkyle-$C_1-C_4$
un groupe furanne-carbonyle, thiophène-carbonyle, pyridinecarbonyle, tétrahydrofurannecarbonyle ou tétrahydrothiophènecarbonyle ou
un reste cyclo-alkylcarbonyle $C_3-C_7$,

caractérisé en ce qu'on transforme par acylation un composé de formule générale Ia

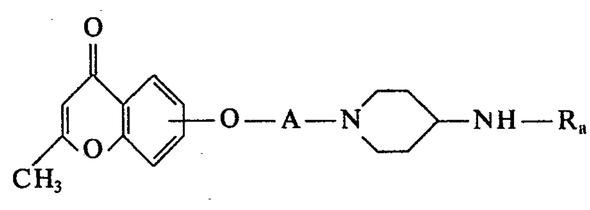
(Ia)

dans laquelle

A est un reste alkylène ayant de 2 à 4 atomes de carbone et,
$R_a$ est de l'hydrogène,

avec un composé R—Z,
dans lequel R a la signification ci-dessus et,
Z est un reste réactif,
on transforme ensuite si on le souhaite le groupe R au moyen d'un procédé connu en un autre groupe R correspondant à la signification donnée ci-dessus
et on transforme, si on le souhaite, le produit de réaction ainsi obtenu en un sel pharmacologiquement compatible.

5. Médicament caractérisé en ce qu'il a une teneur en composés selon la revendication 1 et en substances de support et auxiliaires habituelles.

6. Utilisation de composés selon la revendication 1 pour la fabrication de médicaments ayant des propriétés anti-allergiques.

7. Composés selon la revendication 1 pour le traitement de maladies allergiques.